Europäisches Patentamt
European Patent Office
Office européen des brevets

(19)

(11) Veröffentlichungsnummer: **0 255 664 A2**

(12) # EUROPÄISCHE PATENTANMELDUNG

(21) Anmeldenummer: **87110662.1**

(51) Int. Cl.⁴: **C07C 121/75**

(22) Anmeldetag: **23.07.87**

(30) Priorität: **05.08.86 DE 3626411**

(43) Veröffentlichungstag der Anmeldung: **10.02.88 Patentblatt 88/06**

(84) Benannte Vertragsstaaten: **CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Lantzsch, Reinhard, Dr.**
**Am Buschhäuschen 51**
**D-5600 Wuppertal 1(DE)**
Erfinder: **Krall, Hermann-Dieter, Dr.**
**Irmgardstrasse 8**
**D-4000 Düsseldorf 1(DE)**

(54) **Verfahren zur Herstellung von dimeren aromatischen Acylcyaniden.**

(57) Die vorliegende Erfindung betrifft ein neues Verfahren zur Herstellung von bekannten dimeren aromatischen Acylcyaniden der allgemeinen Formel

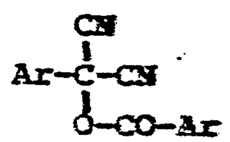

(I)

in welcher

Ar für gegebenenfalls substituiertes Phenyl, Naphthyl oder Hetaryl steht,

welches dadurch gekennzeichnet ist, daß man Säurehalogenide der allgemeinen Formel

Ar-CO-Hal (II)

worin

Ar die oben angegebene Bedeutung besitzt und Hal für Fluor, Chlor oder Brom steht,

mit Alkalicyaniden in einem Zweiphasensystem bestehend aus Wasser und einem mit Wasser nicht oder nur begrenzt mischbaren aliphatischen Keton sowie in Gegenwart eines Phasentransferkatalysators umsetzt.

EP 0 255 664 A2

# Verfahren zur Herstellung von dimeren aromatischen Acylcyaniden

Die vorliegende Erfindung betrifft ein neues Verfahren zur Hestellung von bekannten dimeren aromatischen Acylcyaniden.

Während aliphatische Acylcyanide sehr leicht und zum Teil sogar spontan dimerisieren, sind aromatische Acylcyanide nicht durch Dimerisierung herstellbar. Ihre Herstellung in reiner Form ist bekanntermaßen mit Schwierigkeiten verbunden.

1. Die Umsetzung von aromatischen Säurechloriden mit flüssiger Blausäure und Pyridin ist bekannt; Berichte 41, 1893 (1908).

Die Handhabung der giftigen Blausäure in technischem Maßstab ist jedoch nicht umproblematisch. Ferner ist Pyridin teuer und schwierig wiederzugewinnen. Zudem liegen die erzielten Ausbeuten unter 60 Prozent der Theorie. Gleiches gilt für die Umsetzung von aromat. Säureanhydriden mit Blausäure. (Siehe dazu US-P 3 134 803).

2. Die übliche Synthesemethode für dimere Acylcyanide ist die Umsetzung von Säurechloriden mit Alkalicyaniden in wäßriger Lösung.

Diese Methode ist jedoch nicht generell anwendbar und man erhält die dimeren Acylcyanide meist in schlechten Ausbeuten, da auch monomere Acylcyanide, Carbonsäuren oder Carbonsäureanhydride entstehen können.

Literaturstellen:
Chemistry and Industry 1970, 1408 (Ausbeuten zwischen 20 und 50 %) Tetrahedron Letters 1974, 26, 2275 (Ausbeute maximal 46 %) Synthesis 1983, 8, 637 (Ausbeute maximal 15 %).

In Chemistry and Industry 1970, 1408 wird berichtet, daß nur o-substituierte Säurechloride und unsubstituiertes Benzoylchlorid zu dimeren Acylcyaniden reagieren.

Nach den Angaben in Tetrahedron Letters 1974, 2275 hergestellte dimere Acylcyanide sind stark mit der entsprechenden Benzoesäure verunreinigt.

Es besteht daher ein stark ausgeprägter Bedarf nach technisch praktikablen Verfahren zur Herstellung dimerer aromatischer Acylcyanide.

Es wurde gefunden, daß man dimere aromatische Acylcyanide der allgemeinen Formel

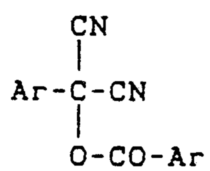

(I)

in welcher

Ar für gegebenenfalls substituiertes Phenyl, Naphthyl oder Hetaryl steht,

erhält, wenn man Säurehalogenide der allgemeinen Formel

Ar-CO-Hal (II)

worin

Ar die oben angegebene Bedeutung besitzt und Hal für Fluor, Chlor oder Brom steht,

mit Alkalicyaniden in einem Zweiphasensystem bestehend aus Wasser und einem mit Wasser nicht oder nur begrenzt mischbaren aliphatischen Keton sowie in Gegenwart eines Phasentransferkatalysators umsetzt.

Es ist als ausgesprochen überraschend zu bezeichnen, daß gemäß der erfindungsgemäßen Umsetzung sehr reine dimere arom. Acylcyanide in hoher Ausbeute anfallen, da z.B. gemäß Tetrahydron Letters 1974, 2275 unter ähnlichen Reaktionsbedingungen nur wesentlich geringere Ausbeuten resultieren und die dimeren Acylcyanide durch die Carbonsäuren verunreinigt nur einen Gehalt von ca. 80-85 % aufweisen. Eine Reinigung gelingt nur schwierig und ist mit großen Verlusten verbunden.

Verwendet man 3,4-Dichlorbenzoylchlorid als Säurehalogenid der Formel (II), Natriumcyanid als Alkalicyanid, Methylisobutylketon als aliphatisches Keton sowie Triethylbenzylammoniumchlorid als Phasentransfer-Katalysator, so kann der Reaktionsverlauf durch folgendes Formelschema wiedergegeben werden:

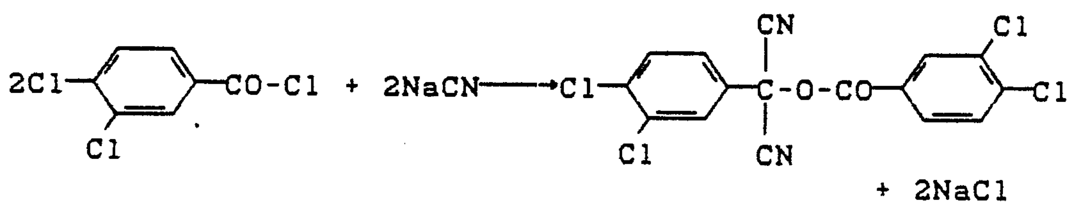

In den Formeln (I) und (II) steht der Substituent Ar vorzugsweise für jeweils substituiertes Phenyl, Naphthyl, Pyridyl, Thiazolyl, Pyrimidinyl, Thienyl oder Furyl.

Als Substituenten für die oben bezeichneten Reste Ar kommen vorzugsweise in Frage:

Halogen (insbesondere Cl, Br, F) Alkyl, Alkoxy, Halogenalkyl, Hydroxy, Halogenalkoxy, Alkylthio, Amino, Halogenalkylthio, Nitro, Cyano, Alkylsulfonyl, Halogenalkylsulfonyl, gegebenenfalls halogensubstituiertes Alkylendioxy, Alkoxyalkyl, Halogenalkoxyalkyl, Carbonyl, Carbalkoxy, der Rest Alkoxy-N = CH-.

Insbesondere kommt für den Substituenten Ar Phenyl oder Pyridyl in Frage, das gleich oder verschieden durch einen oder mehrere der folgenden Reste substituiert ist:

Halogen, Nitro, Amino, CH, OH, $C_{1-4}$-Alkyl, $C_{1-4}$-Halogenalkyl, C $_{1-4}$-Alkoxy, $C_{1-4}$-Halogenalkoxy, Methylendioxy, Ethylendioxy, Difluormethylendioxy, halogensubstituiertes Ethylendioxy, $C_{1-4}$-Alkylthio, $C_{1-4}$-Halogenalkylthio, $C_{2-8}$-Halogenalkoxyalkyl, $C_{1-4}$-Alkylsulfonyl, $C_{1-4}$-Halogenalkylsulfonyl, Carbonyl, Carbalkoxy sowie der Rest $C_{1-4}$-Alkoxy-N = CH-.

In besonderem Maße steht Ar für Phenyl, welches durch Halogen, $C_{1-4}$-Alkyl, $C_{1-4}$-Alkoxy, $C_{1-4}$-Halogenalkoxy, $C_{1-4}$-Halogenalkyl, $NH_2$, $CH_3O$-N = CH-oder $NO_2$ substituiert ist.

Als Alkalicyanide kommen beim erfindungsgemäßen Verfahren vorzugsweise NaCN und KCN in Frage, als aliphatische Ketone kommen vorzugsweise solche mit 4 bis 15 Kohlenstoffatomen in Frage, beispielhaft seien aufgeführt:

Butanon, Methylisopropylketon, Methylpropylketon, Methylbutylketon, Methylisobutylketon, Pinakolin, Diethylketon, Dipropylketon, 5-Methyl-3-heptanon, Diisobutylketon, 2-Undecanon.

Besonders bevorzugtes aliphatisches Keton ist Methylisobutylketon.

Als Phasentransferkatalysatoren kommen insbesondere quartäre Ammoniumsalze in Frage, wie z.B. Tetrabutylammoniumbromid, Triethylbenzylammoniumchlorid, Octyltrimethylammoniumchlorid, Dimethyldodecyl-benzylammoniumchlorid, Tetraethylammoniumchlorid, Tetrabutylammoniumchlorid.

Die Ausgangsverbindungen der Formel (II), die Alkalicyanide sowie die Phasentransferkatalysatoren sind literaturbekannt.

Das erfindungsgemäßen Verfahren wird vorzugsweise wie folgt durchgeführt.

Es werden das Säurechlorid (II) und das quartäre Ammoniumsalz (0,01 bis 10 Mol-%) in dem aliphatischen Keton vorgelegt, und eine wäßrige Alkalicyanidlösung wird zugetropft. Es werden mindestens 1 Mol Cyanid pro Mol Säurehalogenid angewendet, bevorzugt ist ein geringer Überschuß von bis zu 20 % Alkalicyanid.

Das Alkalicyanid wird als möglichst konzentrierte wäßrige Lösung eingesetzt. Verdünnte Lösungen sind jedoch ebenfalls möglich. Bevorzugt sind Konzentrationen zwischen 20 und 40 Mol-%.

Der bevorzugte Temperaturbereich liegt zwischen 0°C und 50°C, besonders bevorzugt zwischen 0°C und 20°C.

Reaktionsdauer: 15 Minuten-12 Stunden, abhängig von der Reaktivität des eingesetzten Säurehalogenids. Die Isolierung des dimeren Acylcyanid der Formel (I) erfolgt durch Filtration bzw. Aufarbeitung der Mutterlauge.

Die Umsetzung kann bei Normaldruck, aber auch bei erhöhtem Druck durchgeführt werden. Im allgemeinen arbeitet man bei Normaldruck.

Die erfindungsgemäß hergestellten Stoffe dienen zur Herstellung von insektiziden und akariziden Hydroxymalonsäurediamiden (vergl. dazu EP-Patent Nr. 76 957).

Weiterhin besitzen die so erhaltenen Hydroxymalonsäurediamide fungizide Wirksamkeit (vgl. Niederländische Anmeldung Nr. 6 704 966).

Beispiel für die Herstellung insektizid und akarizid wirksamer Hydroxymalonsäurediamide aus dimeren aromatischen Acylcyaniden mit Hilfe der sauren Hydrolyse:

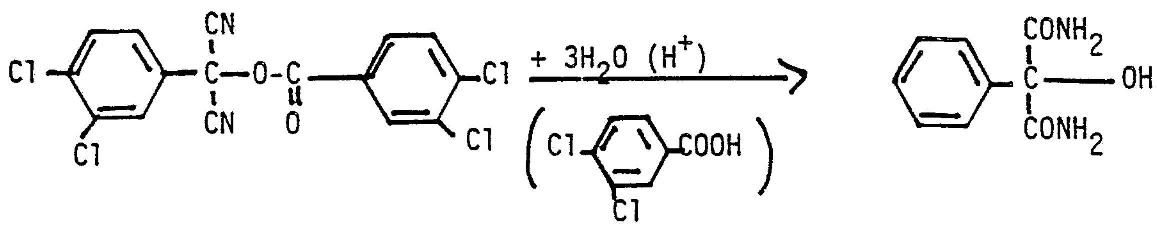


Die anderen Hydroxymalonsäurediamide können entsprechend hergestellt werden, wobei man die entsprechenden dimeren Acylcyanide einsetzt.

Herstellungsbeispiel

Beispiel 1

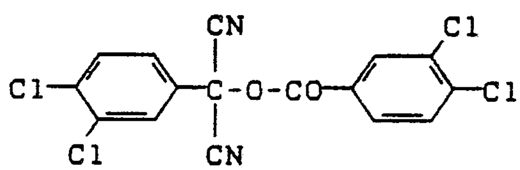

62,8 g (0,3 Mol) 3,4-Dichlorbenzoylchlorid und 300 mg Triethylbenzylammoniumchlorid werden in 250 ml Methylisobutylketon vorgelegt. Bei 25°C werden unter Rühren 15 g (0,308 Mol) Natriumcyanid in 57 ml Wasser zugetropft. Man rührt 10 Stunden bei 20-25°C nach und filtriert ab. Man erhält 86,3 g (71,9 % d. Th.) 3,4-Dichlorbenzoesäure-α,α-bis-cyano-3,4-dichlor-benzylester vom Schmelzpunkt 110°C.

Beispiel 2

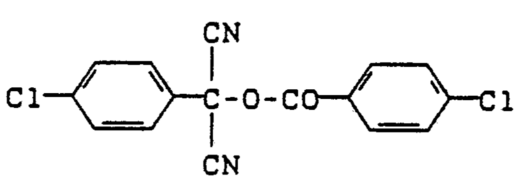

262,5 g (1,5 Mol) 4-Chlorbenzoylchlorid und 2,5 g Tetrabutylammoniumchlorid werden in 600 ml Methylisobutylketon vorgelegt und auf 0°C abgekühlt. Man tropft eine Lösung aus 75 g Natriumcyanid in 225 ml Wasser unter Kühlung so zu, daß eine Temperatur von 5°C nicht überschritten wird. Anschließend gibt man 300 ml Wasser zu, rührt 5 Minuten nach, filtriert und wäscht mit Methylisobutylketon nach. Nach dem Trocknen erhält man 425 g 4-Chlorbenzoesäure-α,α-bis-cyano-4-chlorbenzylester (85,6 % d. Th.). Schmelzpunkt: 158-159°C.

Beispiel 3

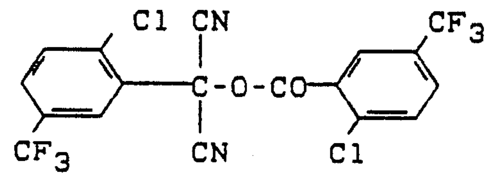

243 g 2-Chlor-5-trifluormethylbenzoylchlorid und 1,8 g Tetrabutylammoniumchlorid werden in 410 ml Butanon vorgelegt und 50,2 g Natriumcyanid in 190 ml Wasser bei Raumtemperatur zugetropft. Man rührt 12 Stunden bei Raumtemperatur nach, saugt dann ab und wäscht mit Butanon nach.

Ausbeute: 166 g 2-Chlor-5-trifluormethylbenzoesäure-α,α-bis-cyano-2-chlor-5-trifluormethylbenzylester (72 % der Theorie); Schmelzpunkt: 130 °C.

Beispiel 4

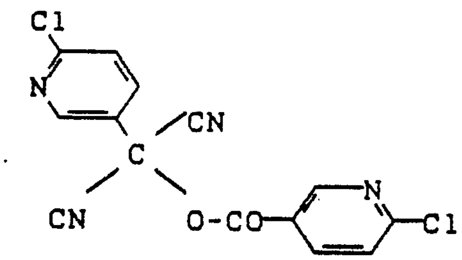

17,6 g (0,1 Mol) 2-Chlor-pyridin-5-carbonsäurechlorid und 0,35 g (1 mMol) Tetrabutylammoniumbromid werden in 80 ml Methylisobutylketon vorgelegt. Dann werden bei 20-25 °C 5 g (0,1024 Mol) Natriumcyanid in 19 ml Wasser zugetropft. Nach 1,5 Stunden beginnt ein Feststoff auszufallen. Nach 6 weiteren Stunden wird abgesaugt und mit 30 ml Methylisobutylketon nachgewaschen. Ausbeute: 13,1 g 2-Chlor-pyridin-5-carbonsäure-dicyano-(2-chlor-5-pyridyl)-methylester (78,7 % d.Th.) vom Schmelzpunkt 158 °C.

Analog Beispiel 1 wurden noch folgende dimeren Acylcyanide synthetisiert:

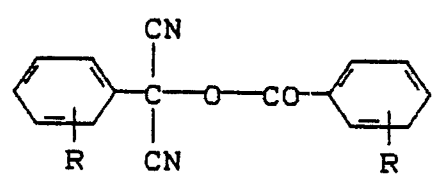

| Beispiel | R | Smp. (°C) | Ausbeute (d.Th.) |
|---|---|---|---|
| 5 | 4-$NO_2$ | 179-180 | 97,8 % |
| 6 | 2-$OCH_3$ | 150-151 | 59,5 % |
| 7 | 4-Br | 153-154 | 87,6 % |
| 8 | 4-$C(CH_3)_3$ | 123 | 54,8 % |

## Ansprüche

1. Verfahren zur Herstellung dimerer aromatischer Acylcyanide der allgemeinen Formel

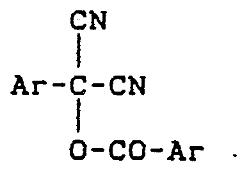

(I)

in welcher

Ar für gegebenenfalls substituiertes Phenyl, Naphthyl oder Hetaryl steht,

dadurch gekennzeichnet, daß man Säurehalogenide der allgemeinen Formel

Ar-CO-Hal (II)

worin Ar die oben angegebene Bedeutung besitzt und

Hal für Fluor, Chlor oder Brom steht,

mit Alkalicyaniden in einem Zweiphasensystem bestehend aus Wasser und einem mit Wasser nicht oder nur begrenzt mischbaren aliphatischen Keton sowie in Gegenwart eines Phasentransferkatalysators umsetzt.

2. Verfahren gemäß Anspruch 1) zur Herstellung von Verbindungen der Formel (I), in welcher

Ar für jeweils durch Halogen, Alkyl, Alkoxy, Halogenalkyl, Hydroxy, Halogenalkoxy, Alkylthio, Amino, Halogenalkylthio, Nitro, Cyano, Alkylsulfonyl, Halogenalkylsulfonyl, gegebenenfalls halogensubstituiertes Alkylendioxy, Alkoxyalkyl, Halogenalkoxyalkyl, Carboxyl, Carbalkoxy, Alkoxy-N=CH-substituiertes Phenyl, Naphthyl, Pyridyl, Thiazolyl, Pyrimidinyl, Thienyl oder Furyl steht.

3. Verfahren gemäß Anspruch 1, dadurch gekennzeichnet, daß als Phasentransferkatalysator ein quartäres Ammoniumsalz eingesetzt wird.

4. Verfahren gemäß Anspruch 1, dadurch gekennzeichnet, daß als Phasentransferkatalysator eine Verbindung der Reihe Tetrabutylammoniumbromid, Triethylbenzylammoniumchlorid, Octyltrimethylammoniumchlorid, Dimethyl-dodecyl-benzylammoniumchlorid, Tetramethylammoniumchlorid, Tetrabutylammoniumchlorid eingesetzt wird.

5. Verfahren gemäß Anspruch 1, dadurch gekennzeichnet, daß als aliphatisches Keton ein solches mit 4 bis 15 Kohlenstoffatomen eingesetzt wird.

6. Verfahren gemäß Anspruch 1, dadurch gekennzeichnet, daß als aliphatisches Keton einer Verbindung der Reihe Butanon, Methylisopropylketon, Methylpropylketon, Methylbutylketon, Methylisobutylketon, Pinakolin, Diethylketon, Dipropylketon, 5-Methyl-3-heptanon, Diisobutylketon, 2-Undecanon eingesetzt wird.

7. Verfahren gemäß Anspruch 1, dadurch gekennzeichnet, daß pro Mol Säurehalogenid (II) 1 bis 1,2 Mol Alkalicyanid eingesetzt wird.

8. Verfahren gemäß Anspruch 1, dadurch gekennzeichnet, daß die Umsetzung in Temperaturbereich zwischen 0°C und 50°C durchgeführt wird.